# FASCICULE DE BREVET EUROPEEN

(11) **EP 3 996 842 B1**
(45) Date de publication et mention de la délivrance du brevet: **02.08.2023**
(21) Numéro de dépôt: 20737193.1
(22) Date de dépôt: 09.07.2020
(51) Int. Cl.: B01L 3/00, C12M 1/32, C12M 1/00

(54) **PLAQUE MULTI-PUITS ET SON PROCÉDÉ DE PRÉPARATION**
PLATTE MIT MEHREREN VERTIEFUNGEN UND VERFAHREN ZU IHRER HERSTELLUNG
MULTI-WELL PLATE AND METHOD FOR PREPARING SAME

(30) Priorité: 12.07.2019 FR 1907886
(43) Date de publication de la demande: 18.05.2022
(73) Titulaire: Centre national de la recherche scientifique, 75016 Paris (FR); COMMISSARIAT À L'ÉNERGIE ATOMIQUE ET AUX ÉNERGIES ALTERNATIVES, 75015 Paris (FR); Université Grenoble Alpes, 38400 Saint-Martin-d'Hères (FR)
(72) Inventeur: NICOLAS, Alice, 38054 Grenoble Cedex 9 (FR); MIGDAL, Camille, 38054 Grenoble Cedex 9 (FR)
(74) Mandataire: Lavoix
(86) Numéro de dépôt international: PCT/EP2020/069358
(87) Numéro de publication internationale: WO 2021/008988

(56) Documents cités:
- FR-A1- 3 061 203
- GB-A- 2 334 581
- US-A1- 2003 031 829
- US-A1- 2018 246 411
- Christal A. Worthen ET AL: "Mechanical Deformation of Cultured Cells with Hydrogels : Methods and Protocols" In: "Methods in Molecular Biology", 24 août 2017 (2017-08-24), Humana Press, New York, NY, US, XP055673224, ISSN: 1064-3745 vol. 1627, pages 245-251, DOI: 10.1007/978-1-4939-7113-8_17, abrégé

## Description

La présente invention concerne une plaque multi-puits, son procédé de préparation et son utilisation pour la culture cellulaire ou pour le criblage de molécule thérapeutiques.

La nécessité de rapprocher les tests *in vitro* de conditions plus physiologiques apparaît dans de nombreux domaines de la biologie/pharmacie. En particulier, une inadaptation à ces conditions est estimée largement responsable de l'échec du transfert vers les phases cliniques de nombreuses molécules identifiées *in vitro,* dû à une analyse erronée des cibles et de leur efficacité ainsi qu'à une sous-évaluation de sa toxicité. Les comportements cellulaires donnant lieu à ces prédictions erronées sont au moins partiellement liés la réalisation de cultures *in vitro* dans des conditions trop éloignées des conditions physiologiques.

Dans ce contexte, il existe un besoin de développer des plaques multi-puits permettant de réaliser des tests dans des conditions les plus proches possibles des conditions physiologiques tout en conservant les avantages des tests avec de telles plaques, à savoir la facilité de mise en oeuvre, la reproductibilité, la grande disponibilité, l'éthique.

Des produits sont aujourd'hui développés qui proposent des environnements biomimétiques remplissant ce cahier des charges : environnements 3D, contraintes géométriques pour forcer la géométrie cellulaire physiologique, chambres de perfusion microfluidique pour mélanger les sécrétions cellulaires et mimer la co-culture, chimie de surface biomimétique pour une culture 2D, support à fond élastique pour mimer les propriétés mécaniques physiologiques.

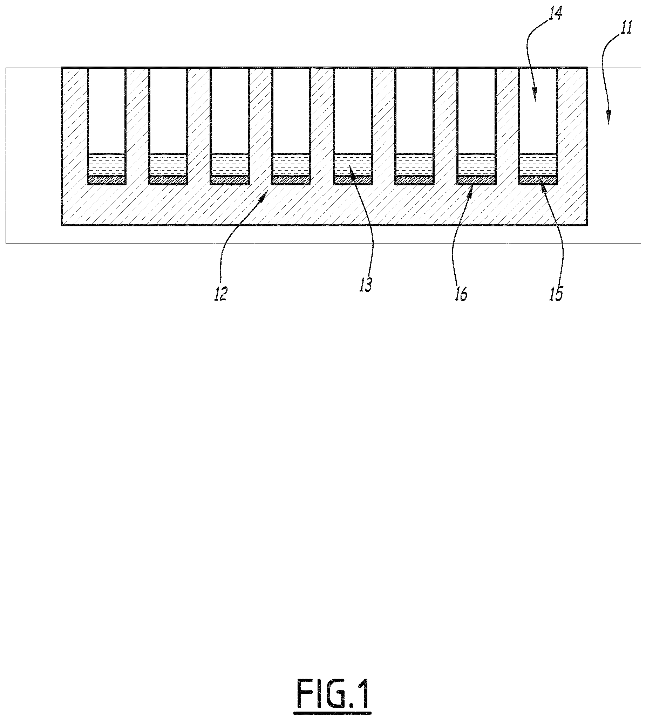

Une méthode pour fabriquer une plaque multi-puits pour laquelle le fond des puits est recouvert d'hydrogel consiste à préparer un film d'hydrogel sur une couche flexible, puis découper l'ensemble film d'hydrogel/ couche flexible sous forme de pastilles et insérer et assembler avec de la colle chaque pastille au fond de chaque puits, comme illustré dans Zustiak, S. et al. Biotechnology and Bioengineering, 2014, 111, 396-403 et à la figure 1. La plaque multi-puits respecte le standard « Recommended Microplate Spécifications » de la Society for Biomolecular Scréening (SBS) et chaque fonds de puits peut avoir une rigidité indépendante de celle des autres fonds de puits. Toutefois, le procédé de préparation requiert énormément de manipulations. Le risque de contamination est très élevé, de même que le risque de dégrader l'état de surface des hydrogels par des résidus de découpe. Enfin, la présence de l'empilement colle+ couche flexible +film d'hydrogel détériore fortement les images obtenues en microscopie inversée.

Alternativement, il est possible de préparer le film d'hydrogel directement au fond des puits d'une plaque multi-puits, comme décrit dans le brevet US 8,871,499. La plaque multi-puits respecte le standard « Recommended Microplate Spécifications » de la Society for Biomolecular Screening (SBS). Cela étant, il est probable que l'épaisseur de gel diffère d'un puits à l'autre. Le brevet rapporte également que les bords du gel de chaque puits sont plus mous. De plus, les résidus de monomères sont très délicats à évacuer du fait de l'étroitesse des puits, en particulier au-delà de 48 puits. Or, ces monomères sont toxiques pour les cellules. Un produit neutralisant les radicaux libres est ajouté, mais avec le risque de conserver une part de toxicité liée aux propriétés mutagènes, cancérigènes et reprotoxiques du bis-acrylamide. Par ailleurs, même s'il est possible de faire varier la rigidité de l'hydrogel entre les puits en faisant varier la composition en monomères de la solution utilisée, la préparation d'hydrogels mous est en pratique délicate car on atteint rapidement la limite à laquelle la réticulation n'est plus covalente mais emmêlée («entanglement»), et le gel présente alors de fortes hétérogénéités spatiales de rigidité. Enfin, cette méthode de préparation interdit de faire des gradients de rigidité contrôlés au sein d'un puits.

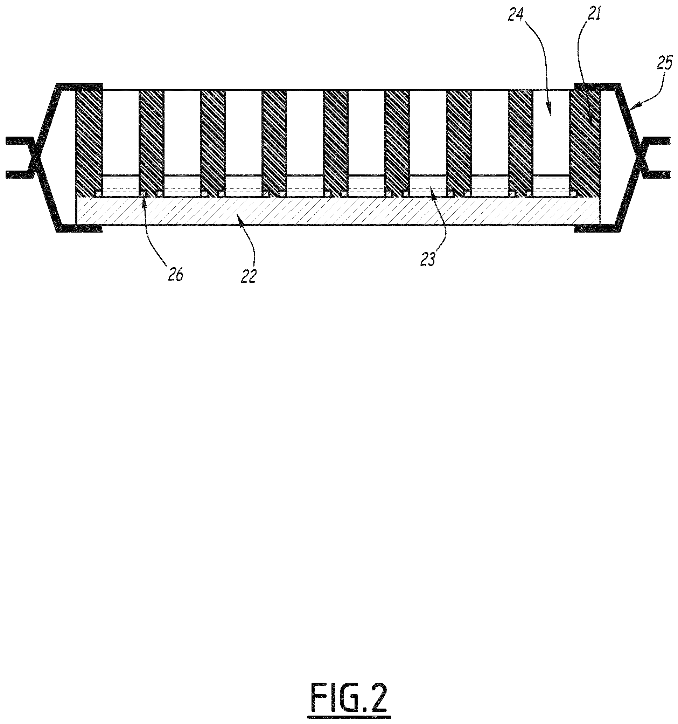

Ahmed, N.; et al. Tissue engineering. Part C, Methods, 2016, 22, 543-551 décrivent la préparation d'une plaque multi-puits comprenant la préparation par photopolymérisation à travers un photomasque d'un hydrogel sous forme de pastilles puis l'assemblage de ces pastilles d'hydrogel avec un support consistant en des puits percés dans une pièce de plastique. L'ensemble est maintenu par des pinces, et des joints annulaires situés à la base de chaque puits assurent l'étanchéité de chaque puits. La figure 2 illustre une telle plaque. La plaque multi-puits obtenue n'est pas compatible avec les robots et les incubateurs de microscope et ne respecte pas le standard « Recommended Microplate Spécifications » de la Society for Biomolecular Screening (SBS), ce qui limite grandement son utilisation. De plus, il est nécessaire de positionner autant de joints que de puits sur la plaque, ce qui rend la méthode très peu adaptée à des plaques comprenant de nombreux puits.

La demande WO 2013/074972 décrit une plateforme pour essais biologiques incluant un substrat sous forme de support (110) dont au moins une surface est revêtue par des marqueurs de position (112), une première couche déformable (120) positionnée au-dessus du substrat, une seconde couche déformable (130) positionnée au-dessus de la première couche déformable, cette seconde couche étant imprégnée de marqueurs de déformation (132). Une couche multi-puits sans fond (150) peut être positionnée au-dessus de la seconde couche déformable, éventuellement par le biais de protéines adhésives Cette demande enseigne que les hydrogels, tels que le polyacrylamide, sont utilisables comme matériaux pour former les couches déformables, mais ont de nombreux inconvénients. Le PDMS serait plus adapté. En outre, cette demande ne décrit pas de colle, et a fortiori pas de colle qui s'étend de la couche multi-puits sans fond jusqu'à la première couche déformable en traversant la seconde couche déformable.

La demande WO 2017/223254 décrit une plaque multi-puits préparée à partir d'agarose ou d'un autre type d'hydrogel. Une plaque 96-puits sans fond est alors comprimée au-dessus de la plaque multi-puits pour créer des macro-puits. Cette demande est également silencieuse sur l'utilisation d'une colle.

La demande WO 2015/061907 décrit un bioréacteur sous forme de plaque multi-puits pour la culture cellulaire, chaque puits comprenant une chambre configurée pour faire de la culture 3D de tissu, et au moins un élément de support déformable fixé à chaque chambre et de préférence polymérique. Dans un mode de réalisation, le bioréacteur comprend un substrat PMMA, des films de polymère (par exemple POMac) en tant qu'élément déformable, une plaque 96 puits sans fond et un revêtement (« cap ») pour la plaque. Des adhésifs double face peuvent être collés à la plaque sans fond. Cela étant, comme explicité ci-après, les inventeurs ont démontré qu'un adhésif ne permet pas de garantir une étanchéité suffisante des puits lorsque le film de polymère est un hydrogel.

La demande GB 2 334 581 décrit une plaque de micro-titration adaptée pour une méthode d'analyse par spectroscopie d'interférence réflectométrie et constituée d'un substrat revêtu de plusieurs couches optiquement actives lié à une plaque multi-puits sans fond au moyen d'un adhésif ou d'un ciment adhésif.

La demande US 2003/031829 décrit une plaque multi-puits pour l'analyse biologique comprenant une plaque multi-puits sans fond et un substrat fixés entre eux au moyen d'un mélange comprenant un adhésif de type NOA-63 et un additif de type silane.

Selon un premier objet, l'invention concerne une plaque multi-puits comprenant un support dont la surface supérieure est recouverte au moins partiellement d'une couche continue d'un hydrogel dont la rigidité, mesurée par microscopie à force atomique, est de 0,05 à 100 kPa et en contact avec la surface inférieure d'une plaque multi-puits sans fond, ledit support, ladite couche continue et ladite plaque multi-puits sans fond étant collés par l'intermédiaire d'une colle qui s'étend d'au moins certaines parties de la surface inférieure de la plaque multi-puits sans fond jusqu'à certaines parties de la surface supérieure du support en traversant la couche continue, le fond de chaque puits de la plaque multi-puits étant totalement entouré par lesdites au moins certaines parties de la surface inférieure de la plaque multi-puits sans fond.

Au sens de la demande, un hydrogel comprend une matrice polymérique et une phase aqueuse au sein de la matrice polymérique, la matrice polymérique formant un réseau tridimensionnel susceptible de gonfler en présence de ladite phase aqueuse. La solubilité de la matrice polymérique à 1 bar et 25°C dans cette phase aqueuse est généralement inférieure à 1 g/L. La phase aqueuse peut être de l'eau.

Le polymère de la matrice polymérique de l'hydrogel peut être homopolymérique (réseau tridimensionnel formé à partir d'un homopolymère), copolymérique (réseau tridimensionnel formé à partir d'un copolymère) ou multipolymérique (réseau tridimensionnel de polymères interpénétrant (« interpenetrating polymeric gel » (IPN) en anglais).

Généralement, la matrice polymérique comprend (voire est constituée de) un polymère choisi parmi :
- les polyacrylamides ;
- les polyéthylènes glycols, polypropylènes glycols et copolymères d'éthylène glycol ou de propylène glycol, ceux-ci comprenant éventuellement des motifs issus de la polymérisation de composés (meth)acrylates;
- les polysaccharides, comprenant éventuellement des motifs répétitifs issus de la polymérisation de composés (meth)acrylates) ;
- les (co)polymères issus de la polymérisation de composés diacrylates et/ou (méth)acrylates ;
- les alcools polyvinyliques comprenant des motifs répétitifs issus de la polymérisation de composés (meth)acrylates;
- les dextranes comprenant des motifs répétitifs issus de la polymérisation de composés (méth)acrylates ;
- les polyfumarates de propylène et les poly (fumarate de propylène-co-éthylène glycol); et
- les combinaisons de ceux-ci.

Les matrices polymériques à base de polyacrylamides, et en particulier issus de la polymérisation de l'acrylamide et du N,N'-méthylènebisacrylamide, sont particulièrement préférées.

Par « composés (meth)acrylates », on entend des composés dérivés d'acrylate ou de méthacrylate, par exemple choisis parmi l'acide acrylique (AA), l'acide méthacrylique (MA), le diméthacrylate d'éthylène glycol (EGDMA), le méthacrylate de 2-hydroxyéthyle (HEMA), l'acrylate de sulfopropyle, où les acides peuvent être sous forme de sel, notamment de sodium ou de potassium.

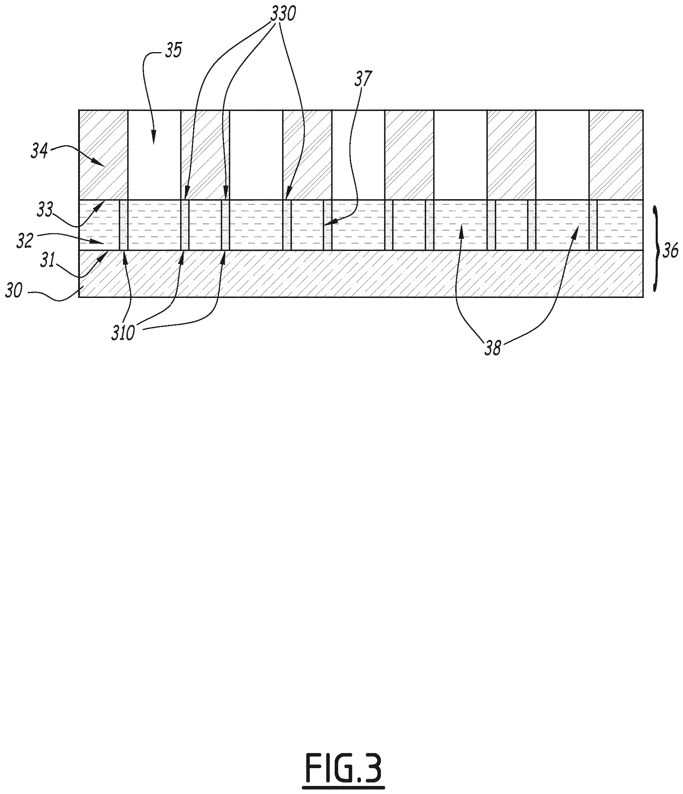

Par « couche d'un hydrogel », on entend que la couche comprend, voire est constituée d'un hydrogel. Cette couche est continue. L'hydrogel forme le fond des puits, et s'étend également entre les puits (figure 3).

La couche continue d'un hydrogel sous forme déshydratée a généralement une épaisseur de 0,1 à 1 mm, typiquement de 0,1 à 200 µm, de préférence de 0,1 à 120 µm, notamment de 10 à 120 µm, par exemple de 20 à 50 µm. L'épaisseur d'un hydrogel sous forme déshydratée est indépendante de sa rigidité, et est donc plus facile à déterminer que celle d'un hydrogel partiellement ou totalement hydraté, car l'épaisseur dépend alors de la rigidité de l'hydrogel.

La rigidité de l'hydrogel est de 0,05 à 100 kPa, de préférence de 0,1 kPa à 50 kPa.

La rigidité locale et donc la variabilité de rigidité sont déterminées par microscopie à force atomique (AFM), par exemple en suivant le protocole décrit pages 29 et 30 de la demande WO 2013/079231.

Au sein d'un même puits, la rigidité de l'hydrogel peut être identique ou variable.

Dans un mode de réalisation, au sein d'un même puits de la plaque multi-puits, la variabilité de la rigidité de l'hydrogel à l'échelle du micromètre est inférieure à 10%, de préférence inférieure à 5%. Autrement dit, la différence de rigidité de deux points de l'hydrogel distants de 1 µm n'excède pas 10%, de préférence 5%. La rigidité de l'hydrogel est alors identique au sein d'un même puits.

Dans un autre mode de réalisation, au sein d'un même puits, l'hydrogel comprend au moins deux zones contiguës de rigidités distinctes présentant un gradient de rigidité supérieur ou égal à 0,05 kPa/µm. La rigidité de l'hydrogel est alors variable au sein d'un même puits. L'hydrogel au sein d'un même puits peut présenter des motifs de rigidité distincts.

La rigidité de l'hydrogel peut être identique ou variable d'un puits à l'autre.

Dans un mode de réalisation, la variabilité de la rigidité de l'hydrogel entre deux puits, de préférence entre tous les puits, est inférieure à 10%, de préférence inférieure à 5%. La rigidité est alors identique entre les deux puits (ou entre tous les puits).

Dans un autre mode de réalisation, la variabilité de la rigidité de l'hydrogel entre au moins deux puits, est supérieure à 20%, de préférence supérieure à 30%. La rigidité est alors variable entre ces deux puits.

La rigidité au sein d'un même puits et entre les puits est indépendante. Par exemple :
- l'hydrogel présente une rigidité uniforme : tous les fonds de puits ont une rigidité identique (c'est-à-dire que la variabilité de la rigidité de l'hydrogel à l'échelle du micromètre est inférieure à 10%, de préférence inférieure à 5%)
- la rigidité de l'hydrogel est identique au sein d'un même puits, mais la rigidité varie d'un puits à l'autre,
- la rigidité de l'hydrogel est variable au sein d'un même puits, et la rigidité varie d'un puits à l'autre.

Ainsi, avantageusement, la rigidité et les propriétés mécaniques de l'hydrogel au sein d'un même puits et entre les puits peuvent être adaptées en fonction de l'utilisation désirée.

La surface de l'hydrogel d'au moins un des puits, de préférence de chaque puits, peut être fonctionnalisée par un polysaccharide et/ou une protéine et/ou un peptide, notamment par une protéine et/ou un peptide induisant une adhésion cellulaire via les intégrines, une telle protéine pouvant être de la fibronectine, du fibrinogène, du collagène, de la laminine, de la vitronectine ou des peptides du type RGD.

Au sens de la demande, par « surface inférieure de la plaque multi-puits sans fond », on entend la surface de la plaque multi-puits qui est en regard ou qui est destinée à être en regard de la couche continue d'un hydrogel. Dans les conditions normales d'utilisation de la plaque multi-puits selon l'invention, la plaque multi-puits selon l'invention, et donc la plaque multi-puits sans fond qu'elle contient, sont à l'horizontale, et la surface inférieure de la plaque multi-puits sans fond est la surface la plus basse de la plaque multi-puits sans fond.

Au sens de la demande, par « surface supérieure du support », on entend la surface du support qui est recouverte ou qui est destinée à être recouverte de la couche continue d'un hydrogel. Dans les conditions normales d'utilisation de la plaque multi-puits selon l'invention, la plaque multi-puits selon l'invention, et donc le support qu'elle contient, sont à l'horizontale, et la surface supérieure du support est la surface la plus haute du support.

Généralement, le support est en verre ou en plastique.

De préférence, l'hydrogel de la couche continue est lié de façon covalente au support.

Par « plaque multi-puits », on entend une plaque comprenant au moins deux puits, typiquement 6, 48, 96, 384 ou 1024 puits.

Les plaques multi-puits sans fond sont des plaques comprenant au moins deux cylindres creux dont les axes sont parallèles les uns aux autres. Ces cylindres forment les puits. Les plaques sont généralement en polymère, par exemple en polystyrène. Les plaques multi-puits sans fond sont disponibles commercialement. On peut par exemple citer les plaques Greiner Bio-One 96 wells No Bottom.

Le support, la couche continue à base d'hydrogel et la plaque multi-puits sans fond sont collés par l'intermédiaire d'une colle qui s'étend d'au moins certaines parties de la surface inférieure de la plaque multi-puits sans fond jusqu'à la surface supérieure du support en traversant la couche continue à base d'hydrogel. Le fond de chaque puits de la plaque multi-puits est totalement entouré par les au moins certaines parties de la surface inférieure de la plaque multi-puits sans fond. La couche continue d'hydrogel est ainsi compartimentée grâce à la colle. La colle permet d'assurer l'étanchéité des puits, c'est-à-dire une étanchéité pendant au moins un mois. Cette étanchéité est généralement observable à l'œil nue. Il est également possible de la tester en introduisant une solution comprenant un colorant dans l'un des puits et de vérifier que celui-ci ne migre pas vers les puits adjacents.

Généralement, chaque zone de la couche continue située en dessous d'un puits de la plaque sans fond a moins de 50% en volume, notamment moins de 25% en volume, de préférence moins de 10% en volume, typiquement moins de 5% en volume, idéalement 0% en volume occupé par de la colle. La partie supérieure d'une de ces zones est le fond d'un puits. On préfère qu'il y ait le moins de colle possible dans l'hydrogel situé au fond de chaque puits afin de ne pas altérer la rigidité de l'hydrogel qui fait le fond du puits. Le volume de chaque zone peut facilement être calculé à partir du rayon des puits et de l'épaisseur de la couche continue. Pour déterminer le volume occupé par la colle dans ces zones, on peut réaliser plusieurs coupes de chaque zone et compiler les résultats. Plus simplement, une observation visuelle de chaque puits permet d'observer à quel point la colle s'étend dans chaque puits.

La colle est de préférence biocompatible et non toxique. On évite généralement les colles à base de cyanolites en raison de leur toxicité. La colle est de préférence résistante à l'ensemble des produits chimiques utilisés lors des analyses (paraformaldéhyde, méthanol par exemple). Il s'agit typiquement d'une colle destinée aux dispositifs médicaux. Des exemples de colles utilisables sont les colles silicone (par exemple la colle Loctite SI5398) ou des colles dérivées de mercapto esters (par exemple les colles Norland Optical Adhesive NOA68 ou NOA81). Il peut s'agir d'une colle obtenue par réticulation, par exemple aux ultraviolets, ou par évaporation de solvant.

Avantageusement, la plaque multi-puits respecte le standard « Recommended Microplate Spécifications » de la Society for Biomolecular Screening SBS qui permet la compatibilité avec les robots et les incubateurs de microscope. En particulier, les caractéristiques physiques des plaques multi-puits sont compatibles avec une utilisation aisée en microscopie à fort grossissement.

De préférence, la plaque multi-puits selon l'invention est susceptible d'être préparée par le procédé décrit ci-après.

Selon un deuxième objet, l'invention concerne un procédé de préparation d'une plaque multi-puits comprenant les étapes consistant à :
a) fournir un substrat comprenant une couche continue d'un hydrogel dont la rigidité, mesurée par microscopie à force atomique, est de 0,05 à 100 kPa, ladite couche recouvrant au moins partiellement la surface supérieure d'un support,
b) appliquer une colle liquide à 20°C sur au moins certaines parties de la surface inférieure d'une plaque multi-puits sans fond, le fond de chaque puits étant totalement entouré par les au moins certaines parties de la surface inférieure de la plaque multi-puits sans fond,
c) assembler le substrat et la plaque multi-puits sans fond en mettant en contact la surface de l'hydrogel du substrat avec la surface inférieure de la plaque multi-puits sans fond au moins partiellement recouverte de colle liquide, ce par quoi la colle liquide pénètre totalement au sein de la couche continue d'un hydrogel.

Le procédé peut comprendre, avant l'étape a), une étape α) de préparation du substrat par polymérisation d'un mélange de monomères susceptibles de former la matrice polymérique de l'hydrogel, la polymérisation étant mise en oeuvre sur le support, ce par quoi est obtenu le support au moins partiellement recouvert d'une couche continue d'hydrogel. La polymérisation peut être une photopolymérisation ou une polymérisation chimique.

Afin de contrôler l'épaisseur de la couche d'hydrogel, on peut réaliser la polymérisation entre le support et une lame faisant office de couvercle, qui sont espacés l'une de l'autre d'une distance égale à l'épaisseur souhaitée, à savoir de préférence de 0,1 à 120 µm, notamment de 40 à 120 µm, par exemple au moyen de cales d'épaisseur.

Lors de l'étape b), la colle liquide est appliquée sur au moins certaines parties de la surface inférieure d'une plaque multi-puits sans fond de manière que le fond de chaque puits soit totalement entouré par les au moins certaines parties de la surface inférieure de la plaque multi-puits sans fond. La colle liquide appliquée entoure donc totalement le fond de chaque puits, ce qui permettra d'assurer l'étanchéité de chaque puits lors des utilisations ultérieures de la plaque multi-puits. Par « fond du puits de la plaque multi-puits sans fond », on entend l'extrémité du puits du côté de la surface inférieure de la plaque multi-puits sans fond.

La(es) forme(s) des au moins certaines parties de la surface inférieure de la plaque multi-puits sans fond sur lesquelles la colle est appliquée est assez diverse.

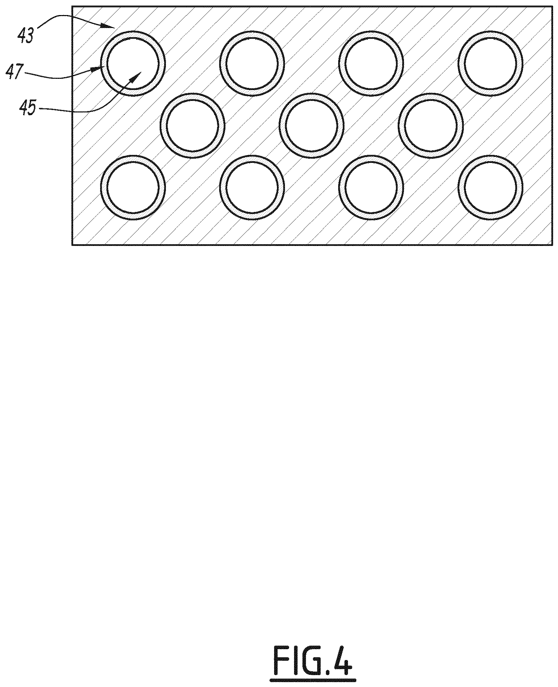

Dans un premier mode de réalisation, la colle liquide est appliquée sur la surface inférieure de la plaque multi-puits sans fond sous forme de cercles, le fond de chaque puits étant totalement entouré par un cercle (figure 4). Les puits de la plaque multi-puits sans fond étant cylindriques, et le fond de chaque puits forme un cercle. Chaque cercle de colle liquide entourant totalement le fond d'un puits peut toucher ou non le cercle formé par le fond du puits.

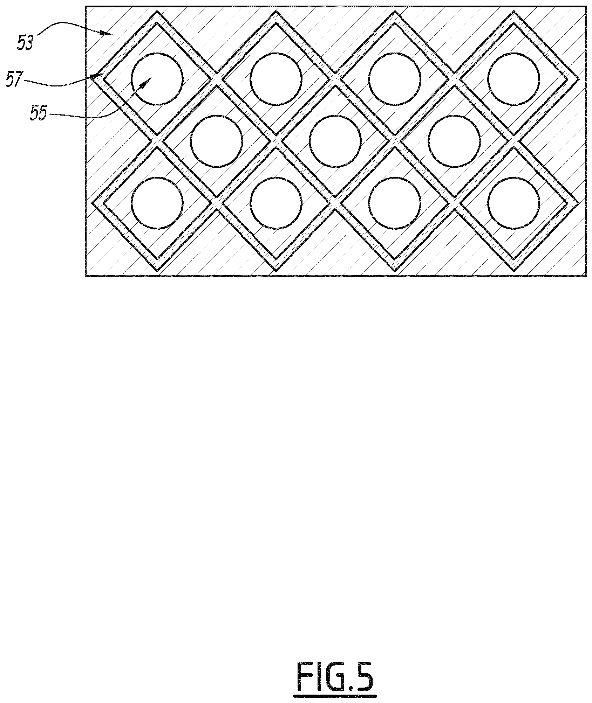

Dans un deuxième mode de réalisation, la colle liquide est appliquée sur la surface inférieure de la plaque multi-puits sans fond sous forme de quadrillage, chaque fond de puits étant totalement entouré par un carreau du quadrillage (figure 5). Un carreau du quadrillage peut être un carré, un rectangle ou un losange selon l'agencement des puits les uns par rapport aux autres dans la plaque multi-puits sans fond.

Ces deux modes de réalisation sont avantageux en ce qu'ils permettent de minimiser la quantité de colle à utiliser.

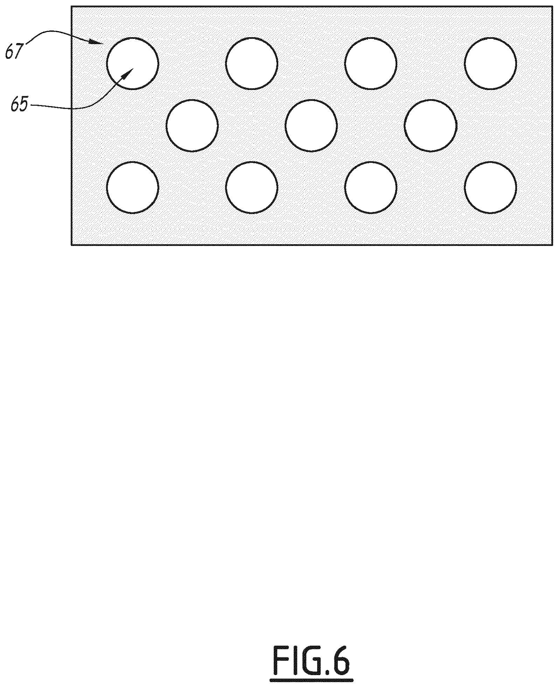

Dans un troisième mode de réalisation, la colle liquide est appliquée sur la totalité de la surface inférieure de la plaque multi-puits sans fond (figure 6). Ce mode de réalisation est facile à mettre en oeuvre, par exemple en passant un rouleau sur la surface inférieure, ou bien en mettant en contact la totalité de la surface inférieure avec un fil de colle, mais c'est celui qui nécessite le plus de colle liquide.

Dans le procédé, la colle liquide est appliquée sur au moins certaines parties de la surface inférieure d'une plaque multi-puits sans fond. En alternative, il serait possible d'appliquer la colle liquide sur au moins certaines parties de la surface de la couche continue d'hydrogel. Cela étant, il faudrait alors mettre le moins de colle possible à la surface de la couche continue qui sera au niveau des puits. Il est plus simple d'appliquer la colle liquide à la surface inférieure de la plaque multi-puits sans fond afin de minimiser la quantité de colle au niveau de chaque puits. De préférence, après l'étape c) d'assemblage, chaque zone de la couche continue située en dessous d'un puits de la plaque sans fond a moins de 50% en volume, notamment moins de 25% en volume, de préférence moins de 10% en volume, typiquement moins de 5% en volume occupé par la colle liquide.

Le procédé peut comprendre avant l'étape c), une étape c0) de déshydratation partielle de l'hydrogel, par exemple par chauffage du substrat entre 30 et 50°C, typiquement pendant 1h à 6 heures. Cette déshydratation permet que la surface de l'hydrogel adhère mieux à la surface inférieure de la plaque multi-puits sans fond au moins partiellement recouverte de colle lors de l'étape c).

Lors de l'étape c), le substrat et la plaque multi-puits sans fond sont assemblés en mettant en contact la surface de l'hydrogel du substrat avec la surface inférieure de la plaque multi-puits sans fond au moins partiellement recouverte de colle liquide, ce par quoi la colle liquide pénètre totalement au sein de la couche continue d'un hydrogel.

La colle utilisée à l'étape b) est une colle liquide à 20°C, ce qui permet qu'elle pénètre dans l'hydrogel lors de l'étape c).

Il y avait un préjugé technique à utiliser une colle liquide pour préparer une plaque multipuits. L'homme du métier expert des hydrogels évite généralement d'utiliser une colle liquide, car il craint que celle-ci ne détériore les propriétés physico-chimiques de l'hydrogel. Il y avait une difficulté technique à coller un hydrogel de manière à garantir la résistance de la plaque multi-puits obtenue en évitant le décollement partiel ou total de la plaque multi-puits sans fond et/ou du support lors de chocs ayant lieu lors de manipulation normale humaine et robotisée (notamment lors de la déshydratation/réhydratation de l'hydrogel) et surtout garantissant l'étanchéité inter-puits. En outre, l'homme du métier spécialiste des plaques multi-puits, dont les applications principales sont en biologie, évite également d'utiliser une colle, car il craint que celle-ci ne soit toxique pour les matériaux biologiques qui seront introduits dans les puits. Or, les présents inventeurs ont démontré qu'il est possible d'utiliser une colle liquide pour préparer la plaque multi-puits sans dénaturer l'hydrogel au fond des puits (sauf à créer quelques effets de bords à la périphérie des puits, mais ces effets de bords existent pour toutes plaques multi-puits, quel que soit leur procédé de préparation).

De manière générale, la pénétration de la colle au sein de la couche continue peut être partielle, c'est-à-dire que la colle ne pénètre pas sur la totalité de l'épaisseur de la couche continue à base d'hydrogel, ou totale, c'est-à-dire qu'elle traverse la couche continue à base d'hydrogel jusqu'à atteindre certaines parties de la surface supérieure du support (généralement les parties de la surface supérieure du support situées à la verticale des parties de la surface inférieure de la plaque multi-puits sans fond sur lesquelles la colle a été appliquée). Dans le contexte de l'invention revendiquée, la pénétration de la colle est totale.

Une pénétration totale peut nécessiter d'augmenter la quantité de colle liquide appliquée lors de l'étape b) par rapport à une pénétration partielle. Lorsqu'elle est totale, le procédé permet alors de préparer la plaque multi-puits selon le premier objet de la demande et l'étanchéité des puits de la plaque est améliorée par rapport à une pénétration partielle. Lors d'une pénétration totale de la colle, l'étanchéité des puits est même maintenue même si l'hydrogel subit une étape de déshydratation après l'étape c). Cela étant, dans une configuration ne faisant pas partie de l'invention revendiquée, la quantité de colle, et donc le coût de la plaque, peuvent avantageusement être minimisés lorsque la pénétration de la colle liquide est partielle. Lorsque la plaque multi-puits est utilisée peu après sa préparation et/ou lorsque les conditions d'utilisation sont douces (notamment lorsqu'elles n'induisent pas de déshydratation de l'hydrogel), une pénétration partielle de la colle est suffisante pour garantir l'étanchéité au minimum 12 heures.

Généralement, la colle liquide a une viscosité de 0.1 à 50 Pa.s (100 à 50000 cPs) à 25°C telle que mesurée selon la norme NF EN 12092 d'août 2002. De préférence, on choisit une colle capable de coller à la fois le matériau du support (généralement en verre ou en plastique) et le matériau de la plaque sans fond (généralement en polymère, souvent en polystyrène). Une colle telle que définie ci-dessus est notamment utilisable. De préférence, on choisit une colle dont le monomère (ou les monomères s'il y en a plusieurs) est susceptible de passer à travers les pores de l'hydrogel de la couche continue. La taille des pores est variable d'un hydrogel à l'autre. Cette capacité à passer à travers les pores peut par exemple être mesurée par microscopie d'exclusion de particules (« particle exclusion microscopy » PEM en anglais) en ajoutant une solution comprenant des particules de taille monodisperse et connue comme explicité au paragraphe 2.2 de Uruena et al., Biotribology, 1-2, 2015, 24-29.

Le procédé peut comprendre, après l'étape c), une étape d) d'activation de la colle, généralement par réticulation, par exemple par illumination aux ultraviolets lorsque la colle comprend un polymère photopolymérisable. L'activation de la colle, lorsqu'elle est requise, doit être suffisante. Il suffit généralement de suivre les préconisations du fournisseur de la colle liquide.

Le procédé peut comprendre, après l'étape c), une étape d') de séchage de la colle, en particulier pour évaporer le solvant quand elle en contient un.

Le procédé peut comprendre, entres les étapes a) et c), une étape a1) d'application d'un polysaccharide et/ou d'une protéine et/ou d'un peptide sur au moins une partie de la surface de l'hydrogel du substrat. Alternativement, le procédé peut comprendre, après l'étape c) (ou après l'éventuelle étape d) ou d')), une étape e) d'application d'un polysaccharide et/ou d'une protéine et/ou d'un peptide sur la surface de l'hydrogel d'au moins un puits, de préférence de chaque puits. Cette étape a1) ou e) permettra d'obtenir, à la fin du procédé, une plaque pour laquelle la surface de l'hydrogel d'au moins un des puits est fonctionnalisée par un polysaccharide et/ou une protéine et/ou un peptide, notamment un polysaccharide, une protéine et/ou un peptide induisant une adhésion cellulaire via les intégrines. Les protéines et/ou les peptides et/ou les polysaccharides mis en oeuvre à l'étape a1) ou e) peuvent avoir été modifiés pour qu'ils soient porteurs d'une fonction susceptible de réagir avec la matrice polymérique de l'hydrogel. L'étape a1) ou l'étape e) peut être suivie d'une étape i) de greffage covalent du polysaccharide et/ou de la protéine et/ou du peptide sur l'hydrogel, ce qui permet de l'immobiliser définitivement et d'éviter que le peptide et/ou protéine ne soit éliminé lors d'un rinçage par exemple.

Les modes de réalisation préférentiels décrits ci-dessus pour la plaque multi-puits sont bien sûr applicables au procédé de préparation d'une plaque.

Le procédé selon l'invention est facile à mettre en oeuvre. Il ne nécessite pas d'équipement complexe. Il est peu coûteux, et facilement reproductible. Le procédé permet de concevoir une plaque 6 puits comme une plaque 384 puits avec des conditions similaires.

Selon un troisième objet, l'invention concerne l'utilisation de la plaque multi-puits décrite ci-dessus pour la culture cellulaire *in vitro,* notamment la culture et la différenciation de cellules souches ou pour le criblage de molécules thérapeutiques.

De préférence, les cellules souches ne sont pas des cellules souches embryonnaires humaines.

L'invention concerne également un procédé de culture cellulaire dans lequel on ensemence la plaque multi-puits décrite ci-dessus avec des cellules, et on cultive les cellules.

L'invention concerne également un procédé de criblage de molécules thérapeutiques comprenant le dépôt dans les puits de la plaque multi-puits décrite ci-dessus d'un ligand, la mise en contact de molécules à tester avec ledit ligand, puis l'identification des molécules à tester s'étant liées au dit ligand. Le ligand peut être un récepteur, un acide nucléique, un peptide ou une protéine.

Les exemples et les figures ci-après illustrent l'invention.

### Figures :

Figure 1 : Schéma d'une vue en coupe d'une plaque multi-puits selon Zustiak (comparatif). Des découpes sous forme de pastilles d'un ensemble film d'hydrogel 13 / couche flexible 15 sont assemblées avec de la colle 16 au fond de chaque puits 14 d'un support 12 d'une plaque multi-puits 11 aux dimensions standardisées.
Figure 2 : Schéma d'une vue en coupe d'une plaque multi-puits selon Ahmed (comparatif). Des pastilles d'hydrogel 23 sont assemblées avec un support consistant en d'une part une pièce de plastique 21 percée de puits 24 et d'autre part une plaque 22 en verre ou en plastique recouverte d'un film de plastique. L'ensemble pièce de plastique 21 / plaque 22 est maintenu par des pinces 25. Des joints annulaires 26 situés à la base de chaque puits 24 assurent l'étanchéité de chaque puits 24.
Figure 3 : Schéma d'une vue en coupe d'une plaque multi-puits selon l'invention. Un substrat 36 comprend un support 30 dont la surface supérieure 31 est recouverte d'une couche continue 32 d'un hydrogel en contact avec la surface inférieure 33 d'une plaque multi-puits sans fond 34. Cinq puits 35 de la plaque multi-puits sans fond 34 sont visibles. Le support 30, la couche continue 32 et la plaque multi-puits sans fond 34 sont collés par l'intermédiaire d'une colle 37 qui s'étend de certaines parties 330 de la surface inférieure 33 de la plaque multi-puits sans fond 34 jusqu'à certaines parties 310 de la surface supérieure 31 du support 30 en traversant la couche continue 32. Des zones 38 de couche continue d'hydrogel sont situées sous les puits de la plaque sans fond. La figure 3 montre un cas idéal où ces zones sont exemptes de colle et où il n'y a pas de colle au fond des puits (chaque zone de la couche continue située en dessous d'un puits de la plaque sans fond a 0% en volume, occupé par de la colle).
Figure 4 : Schéma d'une vue du dessous de la plaque multi-puits sans fond obtenue à la fin de l'étape b) lorsque la colle liquide 47 a été appliquée sous forme de cercles sur la surface inférieure 43 de la plaque multi-puits sans fond, le fond de chaque puits 45 étant entouré par un cercle de colle liquide. Sur cette figure, le cercle de colle liquide touche le cercle formé par le fond du puits.
Figure 5 : Schéma d'une vue du dessous de la plaque multi-puits sans fond obtenue à la fin de l'étape b) lorsque la colle liquide 57 a été appliquée sous forme de quadrillage sur la surface inférieure 53 de la plaque multi-puits sans fond, le fond de chaque puits 55 étant totalement entouré par un carreau du quadrillage.
Figure 6 : Schéma d'une vue du dessous de la plaque multi-puits sans fond obtenue à la fin de l'étape b) lorsque la colle liquide 67 a été appliquée sur la totalité de la surface inférieure de la plaque multi-puits sans fond (qui est donc totalement recouverte de colle liquide 67 et n'est plus visible, sauf si la colle est transparente).

### Exemples :

### Exemple 1 : préparation d'une plaque 96 puits présentant un fond de rigidité de 25 kPa

L'hydrogel consiste en une couche de polyacrylamide, d'une épaisseur d'environ 60 µm. Il a été préparé selon le procédé décrit dans la demande WO 2013/079231. Sa préparation consiste en la réticulation d'une solution photosensible de monomères entre une lamelle de verre basale, qui sert de support à l'hydrogel, et un masque, qui assure la planéité de la surface libre et permet éventuellement d'imprimer des modulations de rigidité si on module son taux de transmission des UV.

### Préparation de la lamelle basale

Une lamelle de verre de dimension 75 × 113 mm² est nettoyée dans une solution de 0.1 mol/L de soude pendant 10 min. Elle est ensuite rincée intensivement à l'eau, puis à l'éthanol, et séchée à l'air. 3000 µl d'une solution de silane comprenant 56 µl de Bind-Silane (GE Healthcare), 485 µl d'acide acétique à 10%, et 14,46 mL d'éthanol ultra pur sont déposés sur la lamelle et frottés avec un chiffon tricoté de polyester jusqu'à disparition des traces de solution. On obtient ainsi une lamelle de verre présentant des fonctions aldéhydes à sa surface, qui permettra le greffage covalent du gel de polyacrylamide.

### Préparation du masque

Un masque transparent en verre permettant de rendre plane la surface de l'hydrogel est traité par un silane fluoré pour limiter son accroche à l'hydrogel : le masque (100 × 110 mm²) est lavé dans une solution d'eau oxygénée/acide sulfurique concentré en proportions 1:2, pendant 10 minutes. Sa surface est ensuite rendue hydrophobe par un traitement Optool (Daikin DSX) : immersion pendant 1 minute dans l'Optool dilué à 1/ 1000 dans du perfluorohexane, puis 1 heure dans de la vapeur d'eau à 80°C, puis immersion sous agitation lente pendant 10 minutes dans du perfluorohexane.

### Préparation de l'hydrogel (étape α) du procédé)

### Composition :

- 10% acrylamide (250 µl de solution initialement à 40%)
- 0.5% N,N'-méthylènebisacrylamide (Bis) (250 µl de solution initialement à 2%)
- 0.2% Irgacure 819 w/v (Ciba, photo-initiateur)
- 1% propylamine (amorceur)
- eau déionisée (490 µl)

L'Irgacure 819 est pesé dans un flacon opaque aux UV. On y ajoute la propylamine. L'ensemble est chauffé à 50°C pendant 2 minutes. Après chauffage, on obtient une solution homogène, transparente. L'eau, l'acrylamide, et le bis acrylamide sont ajoutés rapidement. L'ensemble est homogénéisé doucement à la pipette, pour limiter l'incorporation d'oxygène.

400 µL sont déposés sur la lamelle de verre prétraitée selon le protocole ci-dessus. La lamelle est placée sur un porte-échantillon possédant des cales d'épaisseur qui maintiennent un espacement de 40 µm entre la lamelle et le masque transparent, déposé sur les cales d'épaisseur. L'ensemble (masque, solution, lamelle) est illuminé à l'aide d'un insolateur UV (UVKub Kloé 1) (40 mW/cm² at 365 nm) pendant 15s. Cet ensemble est ensuite plongé dans de l'eau déionisée pour détacher le masque de l'hydrogel à l'aide d'une pince. L'hydrogel est rincé 3 fois avec de l'eau déionisée et conservé dans l'eau déionisée.

### Caractérisation

La rigidité locale du gel est mesurée à l'aide d'un AFM en milieu aqueux (marque JPK). La résistance du gel à l'enfoncement de la pointe est enregistrée. Trente-huit régions de 100 µm x 100 µm distribuées aléatoirement sur la surface de l'hydrogel sont scannées. Les scans sont réalisés avec un pas de 10 µm. Il en résulte une série de courbes d'indentation. Chaque courbe est traitée selon le protocole du fabricant avec un modèle d'indentation élastique. Il en résulte un module d'Young de 25.7 ± 1.7 kPa.

### Assemblage de la plaque 96 puits

L'hydrogel est déshydraté sur une platine chauffante à 37°C pendant 1h.

Un film de colle (Norland Adhesive 68) est déposé sur la surface inférieure d'une plaque 96 puits sans fond (Greiner Bio One 96 wells No Bottom), de telle sorte que la colle entoure totalement chaque puits (étape b) du procédé). L'hydrogel est délicatement déposé sur la surface encollée de la plaque 96 puits (étape c) du procédé). La colle est laissée s'étaler pendant 1 minute. L'ensemble est fixé par illumination UV (5min, lampe UVKub Kloé 1, 40mW/cm²) (étape d) du procédé).

### Fonctionnalisation de surface (étape e) du procédé)

Pour permettre la culture cellulaire, la surface de l'hydrogel est fonctionnalisée avec une protéine de l'adhésion cellulaire, ici la fibronectine.

La protéine fibronectine est préalablement couplée au réticulant hétéro-bifonctionnel sulfo-NHS-LC-Diazirine (Sulfosuccinimidyl-6-(4,4'-azipentanamido)hexanoate, ThermoScientific Pierce; nom commercial: sulfo-LC-SDA), avec un ratio molaire de 1/480.

1 mg de fibronectine (Roche) sont dissous dans 400 µL d'eau déionisée ultrapure, à 37°C pendant 30 min. 0,3 mg mg de sulfo-LC-SDA sont pesés à l'abri de la lumière et dissous clans la solution de fibronectine pendant 30 min à température ambiante. Cette opération est répétée une seconde fois avec 0,2 mg de sulfo-LC-SDA, conduisant au rapport molaire de 1/480. Ce protocole permet de faire réagir la fonction sulfo-NHS du sulfo-LC-SDA avec les groupements amine de la fibronectine en limitant l'hydrolyse du sulfo-LC-SDA.

Le composé formé est une molécule de fibronectine couplée à une fonction photosensible diazirine.

Le composé formé est dialysé à travers une membrane 6-8000 en chambre noire et à 4°C contre 2L de PBS+/+ 1x pendant 48h avec un changement du PBS au bout de 24h. Il est ensuite aliquoté en petits volumes (25 et 50 µL) et conservé congelé à -20°C.

Dans une pièce à éclairage sans UV, 8 mL de solution de fibronectine conjuguée selon le protocole ci- dessus est préparée à une concentration de 8.1 µg/mL dans de l'eau stérile déionisée. 50 µL de cette solution sont déposés à l'aide d'une pipette clans chaque puits de l'assemblage. L'assemblage est placé sur une plaque chauffante à 37°C sous une hotte à flux laminaire jusqu'à évaporation complète de la solution de fibronectine à la surface de l'hydrogel. L'ensemble est ensuite illuminé par la lampe UVKub Kloé 1 pendant 5 min. Chaque puits est ensuite rincé délicatement 3 fois avec une solution de PBS+/+. L'ensemble fonctionnalisé est conservé hydraté dans une solution de PBS+/+, à 4°C, à l'abri de la lumière.

### Culture cellulaire

La lignée cellulaire de glioblastome humain LN229, est ensemencée à raison de 10 000 cellules par puits dans un volume de 100 µl de milieu DMEM (Gibco) supplémenté avec 10% de sérum de veau foetal (PAA) et 1% d'un mélange d'antibiotique et d'antimycotique (ATAM, Thermo Fisher Scientific). Après 24h de culture, le milieu de culture est éliminé par retournement de l'assemblage, et est remplacé pendant 45 min par une solution de fixation (PBS 1X +/+ contenant 4% de paraformaldéhyde).

### Caractérisation des cellules

L'état des cellules est caractérisé par immunofluorescence après 24h de culture. Le marquage de la tubuline, de la vinculine et des noyaux est réalisé comme suit :
Les LN229 de chaque puits sont incubées 15 min dans 100 µl d'une solution de perméabilisation (PBS 1X +/+ contenant 0,5% de Triton X100), puis la solution est éliminée par retournement de l'assemblage et 100 µl de tampon de saturation consistant en une solution de PBS 1X +/+ - Tween20 0.1% - BSA 2%, est ajoutée dans chaque puits pendant 30min sous agitation lente à température ambiante. La solution de saturation est éliminée et remplacée par 50 µl (dans chaque puits) d'une solution de PBS+/+ 1x-Tween20 0.1%-BSA 2% contenant les anticorps monoclonaux primaires anti-tubuline totale (produit chez la souris -YL1/2, Abcam - et dilués au 1/4000) et les anticorps monoclonaux primaires anti-vinculine (produit chez le rat - clone 7F9, Santa Cruz - et dilués au 1/1000).

Les anticorps sont laissés pendant 1h sous agitation lente à température ambiante. La solution d'anticorps primaires est ensuite retirée par retournement et les cellules de chaque puits sont rincées 3 fois par 150 µl de PBS 1X +/+ - Tween20 0.1% - BSA 2%. Les anticorps primaires sont alors révélés avec 50 µl par puits d'une solution de PBS 1X +/+ - Tween20 0.1% - BSA 2% contenant les anticorps secondaires anti-souris couplé à l'Alexa 488 et anti-rat couplé à la Cyanine 3 (respectivement de chez LifeTechnologies et Rockland), pendant 1h sous agitation lente à température ambiante et à l'abri de la lumière. La solution d'anticorps secondaires est retirée et les cellules de chaque puits sont rincées 3 fois par 150 µl de PBS 1X +/+ - Tween20 0.1% - BSA 2%. Puis, pendant 5 min, une solution de PBS 1X +/+ contenant du Hoechst dilué au 1/ 1000 est ajouté à raison de 100 µl/puits. Après élimination de cette solution par retournement, les cellules fixées et marquées sont conservées dans une solution de PBSIX+/+ à 4°C et à l'abri de la lumière.

L'acquisition au 20X et l'analyse des images (9 images par puits) sont réalisées de maniéré automatisée (HCS/HCA High Content Screening/High Content Analysis) par un ArrayScan VTI (Thermo Fisher Scientific). Les résultats démontrent que la culture cellulaire a été efficace dans chaque puits.

### Exemple 2 : Etanchéité des puits selon qu'une colle liquide ou un adhésif est utilisé

Des hydrogels de polyacrylamide fixés sur des lamelles de verre de 30mm sont préparés et sont fixés soit par une colle liquide soit par un adhésif (« colle solide à 20°C ») sous une microplaque sans fond de 96 puits. Entre 7 et 9 puits ont alors un fond en l'hydrogel. Le puits central est rempli d'eau colorée, et l'étanchéité est caractérisée par la diffusion de l'eau colorée vers les puits voisins.

### Fabrication des hydrogels de polyacrylamide

### A Silanisation de la lamelle basale pour le greffage covalent de l'hydrogel de polyacrylamide

Une lamelle de verre basale, de diamètre 30 mm, est nettoyée dans une solution de 0,1 mol/L de soude pendant 10 min. Elle est ensuite rincée intensivement à l'eau, puis à l'éthanol, et séchée à l'air. 500 µl d'une solution de silane comprenant 56 µl de Bind-Silane (GE Healthcare), 484 µl d'acide acétique à 10%, et 14,46 mL d'éthanol ultra pur sont déposés sur la lamelle et frottés avec un chiffon tricoté de polyester jusqu'à disparition des traces de solution. On obtient ainsi une lamelle de verre présentant des fonctions aldéhydes à sa surface, qui permet le greffage covalent du gel de polyacrylamide.

### B Préparation d'une lame anti-adhérente

La polymérisation de l'hydrogel se déroule entre la lamelle silanisée et une lame andi-adhérente qui assure la planéité de la surface de l'hydrogel. Une lame de microscopie optique (26 mm x 76 mm) est lavée dans une solution d'eau oxygénée/acide sulfurique concentré en proportions 1:2, pendant 10 minutes. La lame est rendue anti-adhérente par un traitement hydrophobe d'Optool (Daikin DSX) : immersion pendant 1 minute dans une solution d'Optool dilué à 1/1000 dans du perfluorohexane. Puis la lame est laissée 1 heure dans de la vapeur d'eau à 80°C. Enfin, elle est immergée sous agitation lente pendant 10 minutes dans du perfluorohexane.

### C Préparation de l'hydrogel (étape α) du procédé)

### Composition:

- 10% acrylamide (250 µl de solution initialement à 40%)
- 0.5% N,N'-méthylènebisacrylamide (Bis) (250 µl de solution initialement à 2%)
- 0.2% Irgacure 819 w/v (Ciba, photo-initiateur)
- 1% propylamine (amorceur)
- eau déionisée (490 µl).
L'Irgacure 819 est pesé dans un flacon opaque aux UV. On y ajoute la propylamine. L'ensemble est chauffé à 50°C pendant 2 minutes. Après chauffage, on obtient une solution homogène, transparente. L'eau, l'acrylamide, et le bis acrylamide sont ajoutés rapidement. L'ensemble est homogénéisé doucement à la pipette, pour limiter l'incorporation d'oxygène. 30 µL sont déposés sur la lamelle de verre de 30 mm traitée selon le protocole ci-dessus. La lamelle est placée sur un porte-échantillon possédant des cales d'épaisseur qui maintiennent un espacement de 40 µm entre la lamelle et la lame anti-adhésive, déposée sur les cales d'épaisseur. L'ensemble (lame, solution, lamelle) est illuminé à l'aide d'une lampe fibrée Eleco UVP281 (2 W/cm²) pendant 7 s. Cet ensemble est ensuite plongé dans l'eau pour détacher le masque de l'hydrogel à l'aide d'une pince. L'hydrogel est rincé 3 fois avec de l'eau déionisée et laissé gonfler 1 nuit dans l'eau déionisée. Il est ensuite déshydraté à température ambiante sous une hotte à flux laminaire pendant 4h.

### Collage des hydrogels sous une microplaque 96 puits

Trois colles liquides (invention) et un ruban adhésif double face (comparatif) sont testés :
- Norland NAO 68 (viscosité 5000 mPa.s)
- Norland NOA 81 (viscosité 300 mPa.s)
- Loctite SI5398 (viscosité 20000 mPa.s)
- Ruban double face Tesa^{®} 64621 (90 µm d'épaisseur, sans solvant, caoutchouc synthétique sur support polypropylène).
La face inférieure d'une plaque micropuits sans fond (Greiner Bio-One, ref 655000-06) est nettoyée à l'éthanol 96 % et séchée à l'air.

### A Fixation de l'hydrogel avec une colle liquide (selon l'invention)

Un quadrillage de colle est dessiné sur la face inférieure de la microplaque entre les puits (étape b) du procédé). Le trait de colle est continu, ce qui fait que chaque carré du quadrillage représente une zone continue autour d'un puits de la plaque multi-puits sans fond.

L'hydrogel déshydraté fixé sur la lamelle de verre est posé sur la zone encollée (étape c) du procédé).
- Loctite SI5398 : la lamelle est légèrement pressée contre la microplaque. La colle est laissée sécher pendant 36 h (étape d'))
- NOA 68 et NOA 81 : au bout de 5 min, un examen visuel montre que les colles sont en contact continu avec l'hydrogel suivant le quadrillage dessiné. Les colles sont polymérisés par éclairage UV : lampe fibrée Eleco UVP281 (2 W/cm²) pendant 5 min (étape d) du procédé). Les échantillons sont laissés reposer pendant 36 h.

### B Fixation de l'hydrogel avec le ruban adhésif (comparatif)

L'adhésif est collé sur la face inférieure de la microplaque. Les puits sont découpés à l'aide d'un scalpel. Le film de protection de l'adhésif est retiré et l'hydrogel déshydraté fixé sur la lamelle de verre est mis en contact avec l'adhésif. La lamelle est pressée mécaniquement sur la microplaque avec la main. L'échantillon est laissé reposé pendant 36 h.

### Test d'étanchéité

De l'eau colorée est préparée par la dissolution d'une pointe de spatule de colorant Allura Red AC (Disodium 6-hydroxy-5-[(2-methoxy-5-methyl-4-sulfophenyl)azo]-2-naphthalenesulfonate, 496,42 g/mol) (Sigma, ref 458848) dans de l'eau. 200 µL sont déposés dans le puits central de chacune des conditions. L'ensemble est conservé à 4°C à l'abri de la lumière. Après 72h, les puits voisins sont observés visuellement. Puis le liquide est retiré et la microplaque est observée sur sa face inférieure.

Après observation, 200 µL sont remis dans le puits central et la microplaque est conservée scellée au parafilm pendant 10 jours. Une nouvelle analyse visuelle est alors effectuée.

### Résultats

Les 3 colles liquides offrent des résultats identiques. Au bout de 72h, les puits voisins du puit hydraté sont secs à l'œil, c'est-à-dire ne présentent aucune trace de colorant et ont un indice de réfraction visuellement analogue à celui de l'hydrogel déshydraté. Sur la face inférieure de la plaque, on observe la diffusion du liquide coloré jusqu'à la première barrière de colle mais pas au-delà. Ce résultat est maintenu après 10 jours de conservation.

En revanche, les puits de la plaque obtenue par collage par ruban adhésif double face fuient : au bout de 72h, 2 puits voisins du puits central sont immergés. Sur la face inférieure, on observe que l'ensemble de l'hydrogel a pris la teinte du colorant. Au bout de 10 jours, le puits central est vide, la coloration de l'ensemble de l'hydrogel est plus marquée. Le liquide a fui par les bords de la plaque, distants de plus d'un centimètre des bords du puits central.

### Exemple 3 : Impact de la pénétration partielle ou totale de la colle au sein de la couche continue en hydrogel sur l'étanchéité

L'hydrogel est fabriqué selon le protocole de l'exemple 2 avec la colle Norland NOA68 avec les modifications suivantes:
- lamelle de verre de dimension 113 x 75mm, #1.5 (Live Cell Instrument Co., Ltd, Korea)
- lame anti-adhesive préparée à partir d'une lame de borosilicate de dimension 100 mm (+/-1,0) x 110 mm (+/-1,0) et d'épaisseur 5 mm (+/-0,2) (Prâzisions Glas & Optik GmbH, Germany)
- 300 µL de la solution de monomères sont déposés sur la lamelle silanisée
- la solution est polymérisée pendant 8 s sous une lampe Kloe UV KUB 1 ouverte équipée d'un dépoli (365 nm, 16 mW/cm²).
La face inférieure d'une plaque micropuits sans fond (Greiner Bio-One, ref 655000-06) est nettoyée à l'éthanol 96 % et séchée à l'air. Un quadrillage de colle Norland NOA 68 est dessiné sur la face inférieure de la plaque micropuits entre les puits. Le trait de colle est continu. L'hydrogel déshydraté fixé sur la lamelle de verre est posé sur la zone encollée. L'ensemble est laissé reposé 5 min, temps nécessaire pour observer un changement de l'indice de réfraction à travers la lamelle de verre le long du quadrillage (le contact devient transparent). L'ensemble est ensuite illuminé 5 min à 365 nm (Kloe, 16 mW/cm²). L'ensemble est laissé reposer pendant la nuit à température ambiante. Les puits sont ensuite hydratés par 200 µL d'eau déionisée pendant 24 h. A l'issue de ces 24 h, aucune fuite n'est détectée visuellement.

La plaque micropuits est ensuite déposée sur un bain sec à 37 °C sous une hotte à flux laminaire pendant 24 h pour que l'hydrogel subisse une étape de déshydratation. Le support se décolle par endroit, visible par une variation de l'indice de réfraction sur la face inférieure de la microplaque (perte de la transparence du contact lamelle/microplaque).

Le même protocole mené avec un trait de colle plus épais ayant permis que la colle traverse l'hydrogel et atteigne la lamelle ne conduit pas au décollement du support : l'étanchéité est maintenue même après déshydratation de l'hydrogel.

### Exemple 4 : Impact d'une activation insuffisante de la colle sur l'étanchéité

La plaque micropuits est fabriquée comme à l'exemple 2, avec la modification suivante :
- La réticulation de la colle NOA 68 est effectuée par 1 min d'illumination à 365 nm (Kloe, 16 mW/cm²).
Les puits sont ensuite hydratés par 200 µL d'eau déionisée pendant 3 jours et la face supérieure de la plaque micropuits est scellée avec son couvercle par un ruban de Parafilm M (Brand). A l'issue de ces 3 jours, les puits sont vides. L'eau a fui par les bords du gel.
Cette fuite disparaît avec 5 min d'insolation au lieu de 1 min.

### Exemple 5 : Test de toxicité des colles

### Procédure

Une goutte de colle de 3 mm de diamètre est déposée au fond d'une boite de Pétri traitée culture de diamètre 35 mm (Easy Grip Dish, Corning). Elle est réticulée selon le protocole du fabricant, puis la boite est rincée avec de l'eau désionisée.
Les cellules LN229 (ATCC^{®} CRL-2611) sont ensemencées à une densité de 5000 cellules par cm² et cultivées dans du milieu DMEM GlutaxMax (Gibco, référence ThermoFisher Scientific 31966047) supplémenté de 10 % de FBS (Gibco, référence ThermoFisher Scientific 10270106). L'adhérence et la survie des cellules ainsi que la présence d'une éventuelle contamination sont évaluées après 4h30 et 24h d'ensemencement.

### Résultats selon la nature de la colle

- Colle SuperCyano (Loctite) : après 4h30, les cellules ensemencées n'adhèrent pas au support, contrairement à la boite contrôle qui n'a pas reçu de colle. Dans la boite contrôle, les cellules sont déjà adhérentes et étalées.
- Colle SuperGlue3 (Jelt) : après 4h30, les cellules ensemencées n'adhèrent pas au support, contrairement à la boite contrôle qui n'a pas reçu de colle. Dans la boite contrôle, les cellules sont déjà adhérentes et étalées. Par ailleurs, la colle libère un «voile blanc » dans le milieu de culture.
   Après 24h, toutes les cellules ensemencées avec les colles SuperCyano et SuperGlue3 sont mortes (cellules en suspension), contrairement à la boite contrôle où elles atteignent la confluence.
- Colle NOA68 : après 4h30, les cellules adhèrent et s'étalent de manière analogue au contrôle. Après 24h, les cellules dans la boite contrôle et dans la boite ayant une goutte de NOA68 réticulée se développent de manière analogue (étalement, prolifération).
Aucune des conditions ne présente de contamination (devient rouge si infection fongique, jaune si contamination bactérienne).

## Revendications

1. Plaque multi-puits comprenant un support (30) dont la surface supérieure (31) est recouverte au moins partiellement d'une couche continue (32) d'un hydrogel dont la rigidité, mesurée par microscopie à force atomique, est de 0,05 à 100 kPa, ladite couche continue (32) étant en contact avec la surface inférieure (33) d'une plaque multi-puits sans fond (34), ledit support (30), ladite couche continue (32) et ladite plaque multi-puits sans fond (34) étant collés par l'intermédiaire d'une colle (37), **caractérisée en ce que** la colle (37) s'étend d'au moins certaines parties (330) de la surface inférieure (33) de la plaque multi-puits sans fond (34) jusqu'à certaines parties (310) de la surface supérieure (31) du support (30) en traversant la couche continue (32), le fond de chaque puits (35) de la plaque multi-puits étant totalement entouré par lesdites au moins certaines parties (330) de la surface inférieure (33) de la plaque multi-puits sans fond (34).

2. Plaque multi-puits selon la revendication 1, dans lequel l'hydrogel comprend une matrice polymérique comprenant un polymère choisi parmi :
- les polyacrylamides ;
- les polyéthylènes glycols, polypropylènes glycols et copolymères d'éthylène glycol ou de propylène glycol, ceux-ci comprenant éventuellement des motifs issus de la polymérisation de composés (meth)acrylates;
- les polysaccharides, comprenant éventuellement des motifs répétitifs issus de la polymérisation de composés (meth)acrylates) ;
- les (co)polymères issus de la polymérisation de composés diacrylates et/ou (méth)acrylates ;
- les alcools polyvinyliques comprenant des motifs répétitifs issus de la polymérisation de composés (meth)acrylates;
- les dextranes comprenant des motifs répétitifs issus de la polymérisation de composés (méth)acrylates ;
- les polyfumarates de propylène et les poly (fumarate de propylène-co-éthylène glycol); et
- les combinaisons de ceux-ci,
de préférence un polyacrylamide.

3. Plaque multi-puits selon l'une quelconque des revendications 1 à 2, dans laquelle au sein d'un même puits (35), la variabilité de la rigidité de l'hydrogel à l'échelle du micromètre est inférieure à 10%, de préférence inférieure à 5%.

4. Plaque multi-puits selon l'une quelconque des revendications 1 à 3, dans laquelle, au sein d'un même puits (35), l'hydrogel comprend au moins deux zones contiguës de rigidités distinctes présentant un gradient de rigidité supérieur ou égal à 0,05 kPa/pm.

5. Plaque multi-puits selon l'une quelconque des revendications 1 à 4, dans laquelle la surface de l'hydrogel (32) d'au moins un des puits (35), de préférence de chaque puits (35), est fonctionnalisée par un polysaccharide et/ou une protéine et/ou un peptide, notamment par une protéine et/ou un peptide induisant une adhésion cellulaire via les intégrines, par exemple la fibronectine, le fibrinogène, le collagène, la laminine, la vitronectine ou un peptide RGD.

6. Procédé de préparation d'une plaque multi-puits comprenant les étapes consistant à :
a) fournir un substrat comprenant une couche continue (32) d'un hydrogel dont la rigidité, mesurée par microscopie à force atomique, est de 0,05 à 100 kPa, ladite couche recouvrant au moins partiellement la surface supérieure (31) d'un support (30),
b) appliquer une colle liquide à 20°C sur au moins certaines parties de la surface inférieure (33) d'une plaque multi-puits sans fond (34), le fond de chaque puits (35) de la plaque multi-puits étant totalement entouré par les au moins certaines parties de la surface inférieure (33) de la plaque multi-puits sans fond,
c) assembler le substrat (30) et la plaque multi-puits sans fond (34) en mettant en contact la surface de l'hydrogel du substrat avec la surface inférieure (33) de la plaque multi-puits sans fond (34) au moins partiellement recouverte de colle liquide, ce par quoi la colle (37) liquide pénètre totalement au sein de la couche continue (32) d' hydrogel et la traverse jusqu'à atteindre certaines parties de la surface supérieure (31) du support (30).

7. Procédé selon la revendication 6 , comprenant :
entre les étapes a) et c), une étape a1) d'application d'un polysaccharide et/ou d'une protéine et/ou d'un peptide sur au moins une partie de la surface de l'hydrogel du substrat, ou bien
après l'étape c), une étape e) d'application d'un polysaccharide et/ou d'une protéine et/ou d'un peptide sur la surface de l'hydrogel d'au moins un puits, de préférence de chaque puits.

8. Utilisation d'une plaque multi-puits selon l'une quelconque des revendications 1 à 5 pour la culture cellulaire *in vitro,* notamment la culture et la différenciation de cellules souches ou pour le criblage de molécules thérapeutiques.

## Patentansprüche

1. Multiwell-Platte, umfassend einen Träger (30), dessen obere Oberfläche (31) zumindest teilweise mit einer kontinuierlichen Schicht (32) aus einem Hydrogel bedeckt ist, dessen Steifigkeit, gemessen durch Rasterkraftmikroskopie, 0,05 bis 100 kPa ist, wobei die kontinuierliche Schicht (32) mit der unteren Oberfläche (33) einer bodenlosen Multiwell-Platte (34) in Kontakt ist, wobei der Träger (30), die kontinuierliche Schicht (32) und die bodenlose Multiwell-Platte (34) mittels eines Klebstoffs (37) verklebt sind, **dadurch gekennzeichnet, dass** sich der Klebstoff (37) von mindestens gewissen Abschnitten (330) der unteren Oberfläche (33) der bodenlosen Multiwell-Platte (34) zu gewissen Abschnitten (310) der oberen Oberfläche (31) des Trägers (2) erstreckt und die kontinuierliche Schicht (32) durchdringt, wobei der Boden von jedem Well (35) der Multiwell-Platte vollständig von den mindestens gewissen Abschnitten (330) der unteren Oberfläche (33) der bodenlosen Multiwell-Platte (34) umgeben ist.

2. Multiwell-Platte nach Anspruch 1, wobei das Hydrogel eine Polymermatrix umfasst, umfassend ein Polymer, das ausgewählt ist aus:
- Polyacrylamiden;
- Polyethylenglykolen, Polypropylenglykolen und Copolymeren von Ethylenglykol oder Propylenglykol, diese gegebenenfalls umfassend Einheiten, die aus der Polymerisation von (Meth)acrylatverbindungen stammen;
- Polysacchariden, umfassend gegebenenfalls wiederkehrende Einheiten, die aus der Polymerisation von (Meth)acrylatverbindungen stammen;
- (Co)polymeren, die aus der Polymerisation von Diacrylat- und/oder (Meth)acrylatverbindungen stammen;
- Polyvinylalkoholen, umfassend wiederkehrende Einheiten, die aus der Polymerisation von (Meth)acrylatverbindungen stammen;
- Dextranen, umfassend wiederkehrende Einheiten, die aus der Polymerisation von (Meth)acrylatverbindungen stammen;
- Polypropylenfumaraten und Poly(propylenfumarat-co-ethylenglycol);
und
- Kombinationen davon,
vorzugsweise einem Polyacrylamid.

3. Multiwell-Platte nach einem der Ansprüche 1 bis 2, wobei die Variabilität der Steifigkeit des Hydrogels im Mikrometerbereich innerhalb eines einzelnen Wells (35) weniger als 10 %, vorzugsweise weniger als 5 %, ist.

4. Multiwell-Platte nach einem der Ansprüche 1 bis 3, wobei das Hydrogel innerhalb eines einzelnen Wells (35) mindestens zwei aneinandergrenzende Bereiche mit unterschiedlichen Steifigkeiten umfasst, die einen Steifigkeitsgradienten von 0,05 kPa/pm oder mehr aufweisen.

5. Multiwell-Platte nach einem der Ansprüche 1 bis 4, wobei die Oberfläche des Hydrogels (32) mindestens einer der Wells (35), vorzugsweise jedes Wells (35), mit einem Polysaccharid und/oder einem Protein funktionalisiert ist, insbesondere durch ein Protein und/oder ein Peptid, das eine Zelladhäsion über Integrine, beispielsweise Fibronektin, Fibrinogen, Kollagen, Laminin, Vitronektin oder ein RGD-Peptid induziert.

6. Verfahren zur Herstellung einer Multiwell-Platte, umfassend die Schritte, die aus folgendem bestehen:
a) Bereitstellen eines Substrats, umfassend eine kontinuierliche Schicht (32) aus einem Hydrogel umfasst, dessen Steifigkeit, gemessen durch Rasterkraftmikroskopie, 0,05 bis 100 kPa ist, wobei die Schicht zumindest teilweise die obere Oberfläche (31) eines Trägers (30) bedeckt,
b) Aufbringen eines bei 20 °C flüssigen Klebstoffs auf mindestens Abschnitte der unteren Oberfläche (33) einer bodenlosen Multiwell-Platte (34), wobei der Boden von jedem Well (35) der Multiwell-Platte vollständig von den mindestens gewissen Abschnitten der unteren Oberfläche (33) der bodenlosen Multiwell-Platte umgeben ist,
c) Verbinden des Substrats (30) und der bodenlosen Multiwell-Platte (34) durch Inkontaktbringen der Oberfläche des Hydrogels des Substrats mit der unteren Oberfläche (33) der bodenlosen Multiwell-Platte (34), die zumindest teilweise mit flüssigem Klebstoff bedeckt ist, wobei der flüssige Klebstoff (37) vollständig in die kontinuierliche Hydrogelschicht (32) eindringt und diese durchdringt, bis er Teile der oberen Oberfläche (31) des Trägers (30) erreicht.

7. Verfahren nach Anspruch 6, umfassend:
zwischen den Schritten a) und c) einen Schritt a1) eines Aufbringens eines Polysaccharids und/oder eines Proteins und/oder eines Peptids auf mindestens einen Abschnitt der Oberfläche des Hydrogels des Substrats oder
nach Schritt c) einen Schritt e) des Aufbringens eines Polysaccharids und/oder eines Proteins und/oder eines Peptids auf die Oberfläche des Hydrogels von mindestens einem Well, vorzugsweise jedem Well.

8. Verwendung einer Multiwell-Platte nach einem der Ansprüche 1 bis 5 für die in-vitro-Zellkultur, insbesondere die Kultivierung und Differenzierung von Stammzellen oder zum Screening von therapeutischen Molekülen.

## Claims

1. A multi-well plate comprising a support (30) the upper surface (31) of which is at least partially covered with a continuous layer (32) of a hydrogel which has a stiffness of from 0.05 to 100 kPa, as measured by atomic force microscopy, the said continuous layer (32) being in contact with the lower surface (33) of a bottomless multi-well plate (34), the said support (30), the said continuous layer (32), and the said bottomless multi-well plate (34) being adhered by means of an adhesive (37), **characterised in that** the adhesive (37) extends from at least certain portions (330) of the lower surface (33) of the bottomless multi-well plate ( 34) up to certain portions (310) of the upper surface (31) of the support (30) by passing through the continuous layer (32), the bottom of each well (35) of the multi-well plate being entirely surrounded by the said at least certain portions (330) of the lower surface (33) of the bottomless multi-well plate (34).

2. A multi-well plate according to claim 1, in which the hydrogel comprises a polymer matrix comprising a polymer selected from among:
- polyacrylamides;
- polyethylene glycols, polypropylene glycols and ethylene glycol or propylene glycol copolymers, these latter optionally comprising units resulting from the polymerisation of (meth)acrylate compounds;
- polysaccharides, optionally comprising repeating units resulting from the polymerisation of (meth)acrylate compounds;
- (co)polymers resulting from the polymerisation of diacrylate and/or (meth)acrylate compounds;
- polyvinyl alcohols comprising repeating units resulting from the polymerisation of (meth)acrylate compounds;
- dextrans comprising repeating units resulting from the polymerisation of (meth)acrylate compounds;
- polypropylene fumarates and polypropylene fumarate-co-ethylene glycol); and
- the combinations thereof,
preferably a polyacrylamide.

3. A multi-well plate according to any one of claims 1 to 2, in which within the same given well (35), the variability in the stiffness of the hydrogel at the micrometer scale is less than 10%, preferably less than 5%.

4. A multi-well plate according to any one of claims 1 to 3, in which, within the same given well (35), the hydrogel comprises at least two contiguous zones of distinct stiffness exhibiting a stiffness gradient greater than or equal to 0.05 kPa/µm.

5. A multi-well plate according to any one of claims 1 to 4, in which the surface of the hydrogel (32) of at least one of the wells (35), preferably of each well (35), is functionalised with a polysaccharide and/or a protein and/or a peptide, in particular with a protein and/or a peptide able to induce cell adhesion via integrins, for example fibronectin, fibrinogen, collagen, laminin, vitronectin or an RGD peptide.

6. A plate preparation method for preparing a multi-well plate that comprises the steps consisting in:
a) providing a substrate comprising a continuous layer (32) of a hydrogel which has a stiffness of from 0.05 to 100 kPa, as measured by atomic force microscopy, the said layer at least partially covering the upper surface (31) of a support (30);
b) applying a liquid adhesive at 20°C over at least certain portions of the lower surface (33) of a bottomless multi-well plate (34), the bottom of each well (35) of the multi-well plate being entirely surrounded by the at least certain portions of the lower surface (33) of the bottomless multi-well plate;
c) assembling the substrate (30) and the bottomless multi-well plate (34) by bringing into contact the surface of the hydrogel of the substrate with the lower surface (33) of the bottomless multi-well plate (34) that is at least partially covered with liquid adhesive, whereby the liquid adhesive (37) penetrates totally within the continuous layer (32) of a hydrogel and passes through it until it reaches certain portions of the upper surface (31) of the support (30)..

7. A method according toclaim 6, that includes:
between the steps a) and c), a step a1) of application of a polysaccharide and/or a protein and/or a peptide on to at least a portion of the surface of the hydrogel of the substrate; or else
after the step c), a step e) of application of a polysaccharide and/or a protein and/or a peptide on to the surface of the hydrogel of at least one well, preferably of each well.

8. The use of a multi-well plate according to any one of claims 1 to 5 for *in vitro* cell culture, in particular for the culturing and differentiation of stem cells or for the screening of therapeutic molecules.
